# EUROPEAN PATENT APPLICATION

(11) **EP 3 263 600 A1**
(43) Date of publication of application: **03.01.2018**
(21) Application number: 16305837.3
(22) Date of filing: 01.07.2016
(51) Int. Cl.: C07K 16/28, A01K 67/027, C07K 14/705, A61K 39/00

(54) **INHIBITION OF PLA2R1 FOR THE PREVENTION AND/OR THE TREATMENT OF TYPE 2-DIABETES**

(71) Applicant: Université Claude Bernard Lyon 1, 69100 Villeurbanne (FR); INSTITUT NATIONAL DE LA SANTE ET DE LA RECHERCHE MEDICALE (INSERM), 75013 Paris (FR); Centre Léon Bérard, 69008 Lyon (FR); Centre National de la Recherche Scientifique (C.N.R.S.), 75016 Paris (FR)
(72) Inventor: BERNARD, David, 38460 Trept (FR); VINDRIEUX, David, 42100 Saint-Etienne (FR); GRIVEAU, Audrey, 69008 Lyon (FR); BERTOLINO, Philippe, 69007 Lyon (FR)
(74) Representative: Regimbeau

(57) **Abstract**

Pharmaceutical compositions comprising at least one inhibitor of PLA2R1 and uses thereof in a method for the prevention and/or the treatment of type-2 diabetes.

## Description

The present invention is related to compositions and the use thereof for the prevention and the treatment of a glucose-metabolism disorder and in particular for regulating glycaemia in patients suffering from type-2 diabetes.

Incidence of type-2 diabetes (T2D) is increasing worldwide, in particular due to the increasing of lifespan and food regimen which are more and more lipids- and sugarsenriched.

Type-2 diabetes is associated with other pathologies which may be lethal such as cardiovascular diseases, hypertension, renal disorders, etc. This chronical disease is lowering the lifespan of patients and monitoring and care support of these patients represents a major economical challenge. Compounds which would allow prevention in pre-diabetic or in people risking to develop type-2 diabetes, as well as treatment type-2 diabetes once established is thus of a major interest for patients.

PLA2R1 (Phospholipase A2 receptor 1) is a large glycosylated membrane protein of 180 kDa which is also known as the M-type sPLA2 receptor. It belongs to the superfamily of C-type lectins, and its closest paralogs are the macrophage mannose receptor, the endo-180/UPARAP protein and the DEC-205 (Engelholm et al., 2009; Shrimpton et al., 2009; Taylor et al., 2005). PLA2R1 is a type I receptor comprising a single transmembrane domain, a short cytoplasmic tail, and a very large extracellular region made up of an N-terminal cysteine-rich domain, a fibronectin-like type II domain, and a tandem repeat of eight distinct C-type lectin-like carbohydrate recognition domains (CTLDs). It also exists as a soluble secreted receptor comprising the full extracellular region. PLA2R1 is expressed in several tissues including lung, kidney, spleen and colon. It was originally discovered two decades ago in skeletal muscle cells (M-type receptor) by virtue of binding of various venom and mammalian secreted phospholipases A2 (sPLA2s). Since then, PLA2R1 has been showed to bind extracellular matrix components and sugar linked proteins and participates to cell adhesion. It has also been proposed to mediate or inversely inhibit several *in vitro* functions of the few sPLA2s it can bind and to play a proinflammatory role in a mouse model of septic shock.

We have previously shown that a down-regulation of PLA2R1 favors cellular senescence escape whereas its ectopic expression in normal cells induces cellular senescence (Augert et al., 2009), and in particular favors escape from oncogenic stressinduced senescence (Vindrieux et al., 2013). Our past results also demonstrate that PLA2R1 exerts pro-senescent activities in normal human cells.

Any effect of the receptor PLA2R1 on diabetes and in particular type-2 diabetes was neither described nor suggested.

WO211/094136 and WO2014/151962 described the use of two types of sPLA2 (PLA2G12B and PLA2G12A respectively) for the treatment of glucose-metabolism disorders. However, PLA2R1 is a multifunctional receptor for which the multiple set of *in vivo* biological functions still remain largely unknown mostly due to the diversity of endogenous ligands such as collagen or glycoproteins (Lambeau and Lazdunski, 1999; Murakami et al., 2010). In addition PLA2G12A and PLA2G12B do not bind PLA2R1 suggesting that their effects are unrelated to PLA2R1 (Rouault et al., 2007).

It is now surprisingly shown that the loss of the receptor PLA2R1 improves metabolic functions, leading to a better regulation of glycaemia in response to glucose or insulin injection, which ultimately provides a promising improvement for pathological conditions related to type-2 diabetes.

### Summary

The present invention is related to compositions comprising at least one inhibitor of PLA2R1 for use in a method for the prevention and/or the treatment of type-2 diabetes.

In a preferred embodiment, the compositions of the present invention are for use in a method for the treatment of type-2 diabetes.

Another object of the present invention is a composition comprising at least one inhibitor of the receptor PLA2R1 for use as a medicament.

Preferably, the compositions comprising at least one inhibitor of PLA2R1 which inhibits the expression of the gene encoding the receptor PLA2R1 or inhibit the activity of PLA2R1.

More preferably, the compositions for use as a medicament further comprise another therapeutic agent for the prevention and/or the treatment of type-2 diabetes, and in particular the treatment of type-2 diabetes.

Another object of the present invention is a pharmaceutical composition comprising at least one inhibitor of PLA2R1 and a pharmaceutically acceptable carrier.

In a further object, the present invention is related to compositions comprising at least one inhibitor of PLA2R1 for use in a method for the prevention of obesity.

### Sequence listing

SEQ ID NO: 1: PLA2R1 human nucleotide sequence
SEQ ID NO: 2: PLA2R1 human amino acid sequence

### Detailed description of the invention

The present invention relates to compositions comprising at least one inhibitor of PLA2R1 receptor for use in methods for the prevention and/or the treatment of type-2 diabetes. It has been surprisingly found that loss of PLA2R1 improves metabolic functions, leading to a better regulation of glycaemia in response to *in vivo* glucose or insulin challenges. Therefore, the present invention is directed to compositions comprising at least one inhibitor of PLA2R1 receptor for use in methods for regulation of glycaemia in glucose-metabolism disorders and preferably in type-2 diabetes. The inventors have shown that inhibition of PLA2R1 decreases glucose-resistance, thus maintaining or sustaining glucose-sensitivity during western diets and aging. They have also demonstrated that inhibition of PLA2R1 protects against insulin-resistance and maintains the sensitivity to insulin, thus allowing regulation of glycaemia in response to insulin.

The term "prevention of type-2 diabetes" or "preventing type-2 diabetes" relates to a method for avoiding or delaying the onset of type-2 diabetes in people being prone to develop type-2 diabetes.

The term "inhibitor" according to the present invention refers to a substance, molecule, compound or complex which, directly or indirectly, substantially decreases specifically the expression of the PLA2R1 gene, or the activity and/or the effect of PLA2R1 receptor, for example by competition or conformational inhibition.

In a preferred embodiment, the invention thus relates to a composition comprising at least one inhibitor of PLA2R1 for use in a method for the prevention and/or the treatment of type-2 diabetes wherein said inhibitor substantially decreases specifically the expression of the PLA2R1 gene, or the activity and/or the effect of PLA2R1 receptor.

Preferably said inhibitor specifically inhibits the expression of the PLA2R1 gene, or inhibits the activity and/or the effect of PLA2R1 receptor.

The term "substantially decrease" means any modification of the expression of the gene or activity of PLA2R1 from a significant lowering of the level of the gene expression or activity of PLA2R1 to a complete inhibition of the expression of the gene or activity of PLA2R1, in particular this means a diminution of the gene expression encoding PLA2R1 or of the activity of the PLA2R1 receptor of at least 50%, of at least 60%, of at least 70%, of at least 80%, of at least 90%.

The term "expression" may refer to measuring the level of transcription and/or the level of translation of the gene.

Any classical methods known to the skilled person in the art may be used for determining inhibition of the expression of the PLA2R1 gene, or the activity of the PLA2R1 receptor, such as by detection and quantification of the mRNA of the PLA2R1 gene, by detection and quantification of the polypeptide(s) encoded by the PLA2R1 gene.

The activity of PLA2R1 receptor may be determined indirectly by determining and quantifying the ability to inhibit the phenotypes induced by the PLA2R1 receptor, such as senescence (Augert et al., 2009) or cell death (Augert et al., 2013), or to inhibit the expression of the target genes of PLA2R1 receptor such as the expression of ESRRA (Estrogen Related Receptor Alpha)(Griveau et al., 2016).

The term "PLA2R1" means the receptor PLA2R1 and in particular the human receptor. For example, the polypeptide depicted in SEQ ID NO: 2 is the sequence of the human PLA2R1 receptor encoded by the nucleic sequence depicted in SEQ ID NO: 1.

In another embodiment, the invention relates to a composition comprising at least one inhibitor of PLA2R1 for use in a method for the prevention and/or the treatment of type-2 diabetes wherein said inhibitor is selected from the group consisting of antisense oligonucleotides, interfering RNAs, aptamers, ribozymes avoiding expression of PLA2R1 gene, antagonists of the PLA2R1 receptor activity, antibodies or small molecules including polypeptides of less than 80 residues or pseudopeptides which block or inhibit, directly or indirectly, the activity of PLA2R1 receptor. Said polypeptides inhibitors of the PLA2R1 activity are preferably of at least 5 residues, and more preferably from 8 to 70 residues. The term "pseudopeptides" means a compound comprising, and preferably consisting of, a peptide structure consisting of amino acids and a non-peptide structure.

In a preferred embodiment, the compositions according to the present invention are compositions for use in the prevention and/or the treatment of type-2 diabetes in combination with at least one other therapeutic agent useful for the prevention and/or the treatment of diabetes, preferably of type-2 diabetes.

The present invention encompasses pharmaceutical compositions comprising at least one inhibitor of the receptor PLA2R1 for use in a method for the prevention and/or the treatment of type-2 diabetes, preferably for the treatment of type-2 diabetes.

Another object of the present invention is a composition for use as a medicament comprising at least one inhibitor of the receptor PLA2R1 as described above.

According to a particularly advantageous embodiment, the compositions for use according to the present invention are related to compositions comprising at least one inhibitor of the receptor PLA2R1 as described above for use in a method for the treatment of type-2 diabetes.

Another object of the present invention is a pharmaceutical composition comprising at least one inhibitor of PLA2R1 as described above and a pharmaceutically acceptable carrier.

The present invention also provides pharmaceutical compositions comprising:
a) an effective amount of at least one inhibitor of the receptor PLA2R1 as described herein, and
b) a pharmaceutically acceptable carrier, which may be inert or physiologically active.

As used herein, "pharmaceutically-acceptable carriers" includes any and all solvents, dispersion media, coatings, antibacterial and antifungal agents, and the like that are physiologically compatible. Examples of suitable carriers, diluents and/or excipients include one or more of water, saline, phosphate buffered saline, dextrose, glycerol, ethanol, and the like, as well as combination thereof In many cases, it will be preferable to include isotonic agents, such as sugars, polyalcohols, or sodium chloride in the composition. In particular, relevant examples of suitable carrier include: (1) Dulbecco's phosphate buffered saline, pH ∼ 7.4, containing or not containing about 1 mg/ml to 25 mg/ml human serum albumin, (2) 0.9% saline (0.9% w/v sodium chloride (NaCl)), and (3) 5% (w/v) dextrose; and may also contain an antioxidant such as tryptamine and a stabilizing agent such as Tween 20.

The pharmaceutical compositions encompassed by the present invention may also contain at least one further therapeutic agent useful for the prevention and/or the treatment of diabetes, preferably for the treatment of type-2 diabetes.

Examples of such therapeutic agents which may be combined, alone or in combination, to the inhibitor of PLA2R1 as described herein may be selected among sulfonylureas such as chlorpropamide, glipizide, glyburide and glimepiride; biguanides such as metformin; meglitinides such as repaglinide and nateglinide; thiazolidinediones such as rosiglitazone and pioglitazone; Dipeptidyl peptidase-4 (DDP-4) inhibitors such as sitagiptin, saxagliptin, linagliptin and alogliptin; sodium-glucose transporter 2 (SGLT2) inhibitors such as dapaglifozin and canagliflozin; alpha-glucosidase inhibitors such as acarbose and miglitol; Bile Acid Sequestrants (BAS) such as colesevelam.

The compositions of the invention may be in a variety of forms. These include for example liquid, semi-solid, and solid dosage forms, but the preferred form depends on the intended mode of administration and therapeutic application. Typical preferred compositions are in the form of injectable or infusible solutions. The preferred mode of administration is parenteral (e.g. intravenous, intramuscular, intraperinoneal, subcutaneous). In a preferred embodiment, the compositions of the invention are administered intravenously as a bolus or by continuous infusion over a period of time. In another preferred embodiment, they are injected by intramuscular, subcutaneous, intra-articular, intrasynovial, intratumoral, peritumoral, intralesional, or perilesional routes, to exert local as well as systemic therapeutic effects.

Sterile compositions for parenteral administration can be prepared by incorporating the inhibitor(s) of PLA2R1 as described in the present invention in the required amount in the appropriate solvent, followed by sterilization by micro filtration. As solvent or vehicle, there may be used water, saline, phosphate buffered saline, dextrose, glycerol, ethanol, and the like, as well as combination thereof. In many cases, it will be preferable to include isotonic agents, such as sugars, polyalcohols, or sodium chloride in the composition.

The inhibitor(s) of PLA2R1 as described herein may also be orally administered. As solid compositions for oral administration, tablets, pills, powders (gelatine capsules, sachets) or granules may be used. In these compositions, the active ingredient according to the invention is mixed with one or more inert diluents, such as starch, cellulose, sucrose, lactose or silica, under an argon stream. These compositions may also comprise substances other than diluents, for example one or more lubricants such as magnesium stearate or talc, a coloring, a coating (sugar-coated tablet) or a glaze.

As liquid compositions for oral administration, there may be used pharmaceutically acceptable solutions, suspensions, emulsions, syrups and elixirs containing inert diluents such as water, ethanol, glycerol, vegetable oils or paraffin oil. These compositions may comprise substances other than diluents, for example wetting, sweetening, thickening, flavoring or stabilizing products.

The doses depend on the desired effect, the duration of the treatment and the route of administration used.

In a further object, the present invention is related to compositions comprising at least one inhibitor of PLA2R1 for use in a method for the prevention of obesity.

In a preferred embodiment, said at least one inhibitor of PLA2R1 is selected in the group consisting of antisense oligonucleotides, interfering RNAs, aptamers, ribozymes which avoiding expression of PLA2R1 gene, antagonists of the PLA2R1 receptor activity, antibodies or small molecules including polypeptides of less than 80 residues, preferably of at least 5 residues and more preferably from 8 to 70 residues, or pseudopeptides which block or inhibit, directly or indirectly, the activity of PLA2R1 receptor.

The invention is also related to the use of at least one inhibitor of PLA2R1 as described herein for the manufacture of a medicament for prevention and/or treatment of type-2 diabetes or for prevention of obesity.

The present invention also provides methods for preventing and/or treating type-2 diabetes and methods for preventing obesity including administering an effective amount of at least one inhibitor of PLA2R1 as described herein to a human or to a patient in need thereof. In a preferred embodiment, the invention relates to methods for the treatment of type-2 diabetes.

### Figures

Figure 1:
   A - Gain of weight kinetics in PLA2R1 WT and PLA2R1 KO mice feed with HFHS diet during 84 days (* p<0.05)
   B - Amount of food intake of PLA2R1 WT and PLA2R1 KO mice feed with HFHS diet, measured in grams per mouse per day and calculated during 84 days.
Figure 2:
   A - schematic representation of Intra Peritoneal Glucose Tolerance Test (IPGTT) protocol (eWAT means enteric White Adipose Tissue).
   B - Glycaemia over post-glucose injection in PLA2R1 WT (left panel) and PLA2R1 KO (right panel) mice feed with HFHS diet (42 days after beginning of the HFHS diet: * p<0.05; **p<0.01; *** p<0.005 and 85 days after beginning of the HFHS diet: ° p<0.05; °°p<0.01).

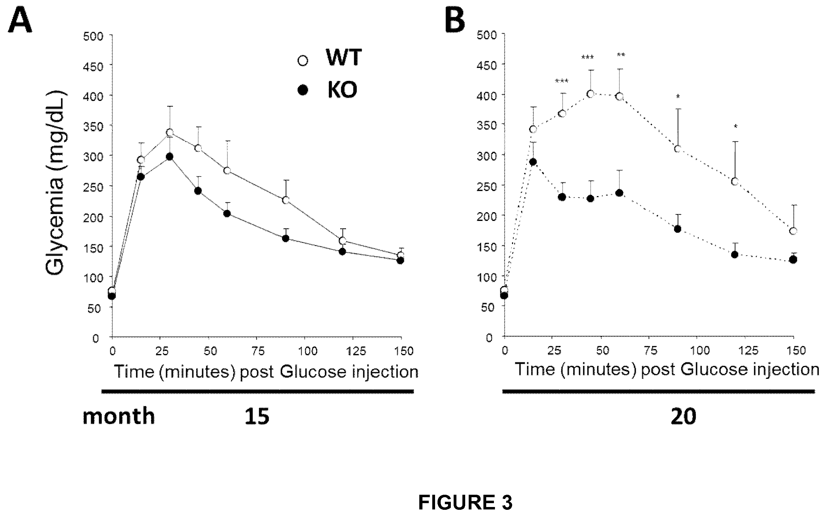

Figure 3: Glycaemia over post-glucose injection in 15-month old (A) and 20-month old (B) PLA2R1 WT and PLA2R1 KO mice feed with normal diet (* p<0.05; ** p<0.01 ; *** p<0.005)
Figure 4:
   A - schematic representation of Intra Peritoneal Insulin Tolerance Test (IPITT) protocol.
   B - Glycaemia over post-insulin injection in PLA2R1 WT (left panel) and PLA2R1 KO (right panel) 20-month old mice feed with normal diet (** p<0.01; *** p<0.005)

### Examples

### MATERIALS AND METHODS

### Mice

C57/B6 PLA2R1 WT and PLA2R1 knockout (KO) littermate mice were generated by crossing C57/B6 PLA2R1 heterozygous mice. The mice were housed in standard cages in an environment with temperature 23±1.5°C, humidity of 55±10% and a 12h dark/light cycle. The animals were allowed ad libitum access to respective diets and water. The animals were handled in accordance with animal care guidelines of the European Union and French laws and were validated by the local Animal Ethic Evaluation Committee (CECCAPP).

### HFHS diet

9-week-old PLA2R1 KO or WT littermate C57BL/6 mice have received during 84 days an Adjusted Calories Diet (42% from Fat) from Harlan (Reference TD.88137) containing Casein (195g/Kg), DL-Methionine (3g/Kg), Sucrose (341.46g/Kg), Corn Starch (150g/Kg), Anhydrous Milkfat (210g/Kg), Cholesterol (1.5g/Kg), Cellulose (50g/Kg), mineral Mix AIN-76 (35g/Kg), Calcium Carbonate (4g/Kg), Vitamin Mix Teklad (10g/Kg) and Ethoxyquin (0.04g/Kg). Mice were weighted once a week during HFHS protocol and the quantity of diet eaten by the mice was checked twice a week.

### IPGTT

The IntraPeritoneal Glucose Tolerance Test measures the clearance of an intraperitoneally injected glucose from the body. Animals were fasted for approximately 16h, in clean cages without diet and feces. Glycaemia was checked just before the glucose injection (2.5g/Kg for the males and 2g/Kg for the females). After glucose injection glycaemia was checked for each animal at different time-points: 15min., 30min., 45min., 60min., 90min., 120min., 150min. and 180min.

### IPITT

The IntraPeritoneal Insulin Tolerance Test determines the sensitivity of insulin-responsive tissues in the rodent. This is determined by measurement of glucose remaining in the circulation over time after a bolus ip insulin injection. Mice were fasted 6h before insulin injection. Glycaemia was measured for each animal just before the injection. The mice had received a dose of 1UI/Kg of insulin and glycaemia were checked at different time-points: 15min., 30min., 45min., 60min, 90min. and 120min.
Glycaemia is measured using Free Style Papillon Easy dosage electrodes (Abbott Laboratories)

### Statistical analysis

Each histograms or points represent the mean±SEM. T-Test was performed for each experiment. * *p*<0.05, ** *p*<0.01, *** *p*<0.005.

### RESULTS

### Loss of PLA2R1 decreases obesity and glucose-resistance during western diets

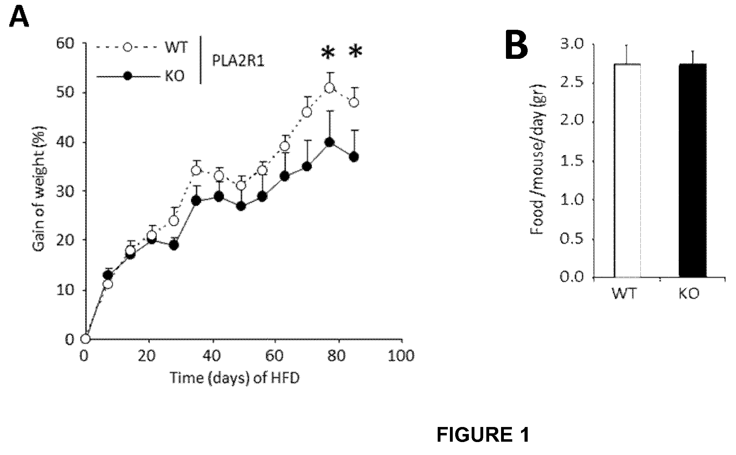

Two month old PLA2R1 WT (n=6) and KO (n=7) mice were feed with HFHS diet and different metabolic parameters were analyzed. PLA2R1 KO mice were found to gain weight slowly when compared to WT mice (Figure 1A) whereas they eat a similar amount of foods (Figure 1B).

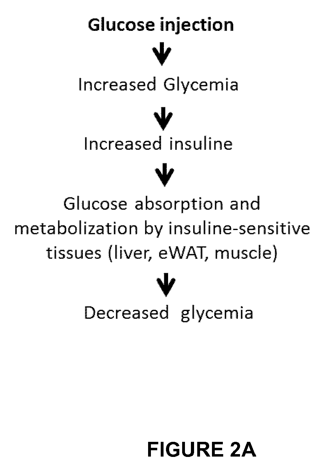

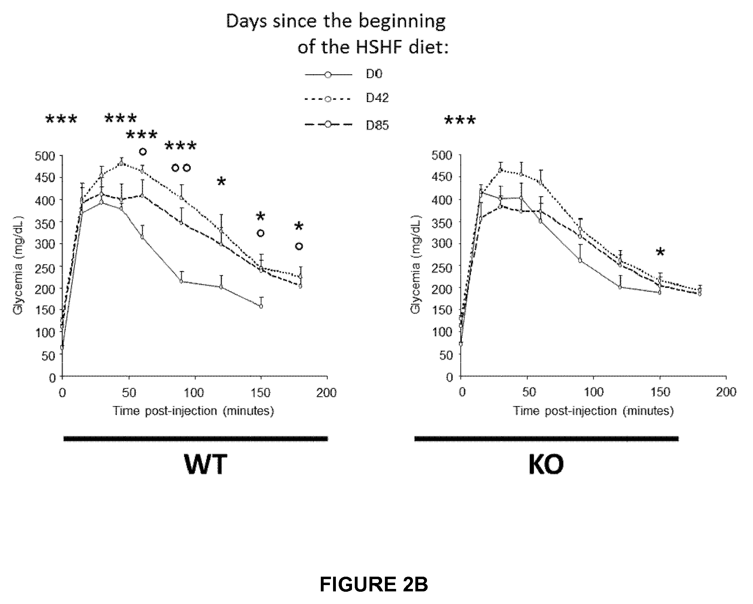

Next, Intraperitoneal Glucose Tolerance Test (IPGTT) was performed, which allow to measure how challenged mice with high glucose was able to restore normal glycaemia (Figure 2A). PLA2R1 WT mice were found to display increased glucose resistance upon the HSHF diet, as expected (Figure 2B, left panel). By contrast, PLA2R1 KO mice maintained their glucose sensitivity during the HSHF diet (Figure 2B, right panel).

These data demonstrate that the loss of PLA2R1 protects against glucose-intolerance that is frequently seen upon the western diet and represent the first clinical signs of a developing T2D (prior the onset of an insulin resistance).

### Loss of PLA2R1 sustains glucose sensitivity during Aging

In human, T2D-like disease incidence strongly increased because of both western diet and increased lifespan. Glucose-intolerance, which appeared during normal aging, was also examined to see if impacted by the loss of function of PLA2R1. IPGTT were performed in PLA2R1 WT (n=7) and KO (n=9) mice fed with normal diet at 15- and 20-month old mice. PLA2R1 WT and KO mice displayed similar glucose sensitivity (Figure 3A). Whereas 20 month old PLA2R1 KO mice sustained glucose sensitivity, the PLA2R1 WT mice displayed glucose-intolerance (Figure 3B). These data demonstrate that loss of function of PLA2R1 sustains glucose sensitivity during aging when compared to WT mice.

### Loss of PLA2R1 protect against insulin-resistance

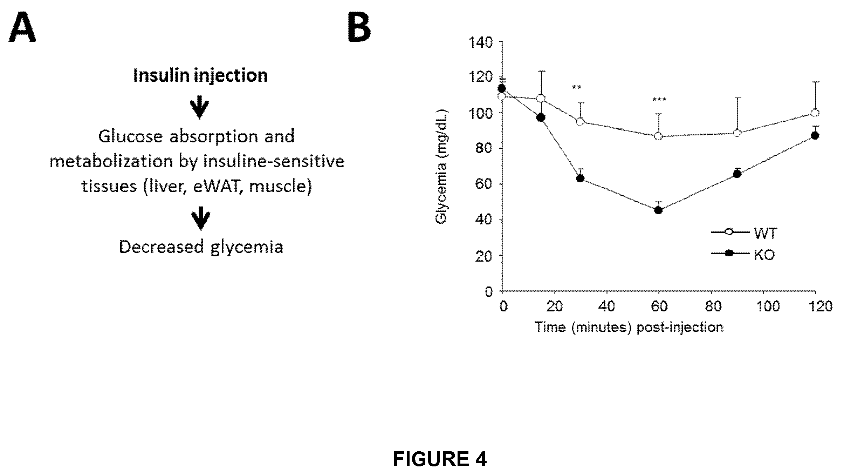

Glucose sensitivity can depend on insulin production as well as on insulin sensitivity (directly relevant for T2D-like disease) of organs metabolizing the glucose. Intraperitoneal Insulin Tolerance Test (IPITT) was then performed to measure the ability of 20 month old PLA2R1 KO mice to respond to increased insulin by measuring glycaemia when compared to PLA2R1 WT mice (Figure 4A). Old PLA2R1 WT mice were insulino-resistant whereas as the PLA2R1 KO mice were still sensitive to insulin as they decreased their glycaemia in response to insulin injection (Figure 4B).

Results from Figures 3 and 4 clearly demonstrate that the impact of the loss of PLA2R1 on glucose sensitivity and insulin resistance is not dependent of the body weight gain sensitivity.

Together, these data demonstrate that loss of function of PLA2R1 improves T2D-like disease and is thus a targetable receptor to prevent and/or to fight T2D.

### REFERENCES

- Augert,A., Payre,C., de Launoit,Y., Gil,J., Lambeau,G., and Bernard,D. (2009). The M-type receptor PLA2R regulates senescence through the p53 pathway. EMBO Rep 10, 271-277.
- Augert A., Vindrieux D., Girard C., Le Calve B., Gras B., Ferrand M., Bouchet B, Puisieux A., De Launoit Y. Simonnet H., Lambeau G. and Bernard D. (2013). PLA2R1 killscancercellsbyinducingmitochondrialstress. Free Radical Biology and Medicine 65, 969-977.
- Engelholm,L.H., Ingvarsen,S., Jurgensen,H.J., Hillig,T., Madsen,D.H., Nielsen,B.S., and Behrendt,N. (2009). The collagen receptor uPARAP/Endo180. Front Biosci. 14, 2103-2114.
- Griveau A., Devailly G., Eberst L., Navaratnam N., Le Calve B., Ferrand M., Faul P., Augert A.,Dante R., Vanacker JM., Vindrieux D. and Bernard D. (2016). The PLA2R1-JAK2 pathway upregulates ERRα and its mitochondrial program to exert tumor-suppressive action. Oncogene, 1-10.
- Lambeau,G. and Lazdunski,M. (1999). Receptors for a growing family of secreted phospholipases A2. Trends Pharmacol. Sci. 20, 162-170.
- Murakami,M., Taketomi,Y., Girard,C., Yamamoto,K., and Lambeau,G. (2010). Emerging roles of secreted phospholipase A2 enzymes: Lessons from transgenic and knockout mice. Biochimie 92, 561-582.
- Rouault M., Le Calvez C., Boilard E., Surrel F., Singer A., Ghomashchi F., Bezzine S., Scarzello S., Bollinger J., Gelb M.H. and Lambeau G. (2007). Recombinant Production and Properties of Binding of the Full Set of Mouse Secreted Phospholipases A2 to the Mouse M-Type Receptor. Biochemistry, 46, 1647-1662.
- Shrimpton,R.E., Butler,M., Morel,A.S., Eren,E., Hue,S.S., and Ritter,M.A. (2009). CD205 (DEC-205): a recognition receptor for apoptotic and necrotic self. Mol. Immunol. 46, 1229-1239.
- Taylor,P.R., Gordon,S., and Martinez-Pomares,L. (2005). The mannose receptor: linking homeostasis and immunity through sugar recognition. Trends Immunol. 26, 104-110.
- Vindrieux,D., Augert,A., Girard,C.A., Gitenay,D., Lallet-Daher,H., Wiel,C., Le,C.B., gras,B., ferrand,M., verbeke,S., de,L.Y., Leroy,X., Puisieux,A., Aubert,S., Perrais,M., Gelb,M., Simonnet,H., Lambeau,G., and Bernard,D. (2013). PLA2R1 mediates tumor suppression by activating JAK2. Cancer Res. 73, 6334-6345.

## Claims

1. Composition comprising at least one inhibitor of the receptor PLA2R1 for use in a method for the prevention and/or the treatment of type-2 diabetes.

2. Composition according to claim 1 for use in a method for the prevention and/or the treatment of type-2 diabetes wherein the inhibitor substantially decreases specifically the expression of the PLA2R1 gene, or the activity and/or the effect of PLA2R1 receptor.

3. Composition according to claim 2 for use in a method for the prevention and/or the treatment of type-2 diabetes wherein the inhibitor inhibits the expression of the PLA2R1 gene, or inhibits the activity and/or the effect of PLA2R1 receptor.

4. Composition according to anyone of claims 1 to 3 for use in a method for the prevention and/or the treatment of type-2 diabetes wherein the inhibitor is selected in the group consisting of antisense oligonucleotides, or interfering RNAs, aptamers, ribozymes avoiding expression of PLA2R1 gene, antagonists of the PLA2R1 receptor activity or expression, antibodies or small molecules including polypeptides of less than 80 residues or pseudopeptides which block or inhibit, directly or indirectly, the activity or the expression of PLA2R1 receptor.

5. Composition according to anyone of claims 1 to 4 for use in a method for the prevention and/or the treatment of type-2 diabetes in combination with at least one other therapeutic agent useful for the prevention and/or the treatment of diabetes, and preferably of type-2 diabetes.

6. Composition according to anyone of claims 1 to 5 for use in a method for the treatment of type-2 diabetes.

7. Composition comprising at least one inhibitor of the receptor PLA2R1 for use as a medicament.

8. Composition for use a medicament according to claim 7 wherein said inhibitor is selected in the group consisting of of antisense oligonucleotides, or interfering RNAs, aptamers, ribozymes avoiding expression of PLA2R1 gene, antagonists of the PLA2R1 receptor activity or expression, antibodies or small molecules including polypeptides of less than 80 residues or pseudopeptides, which block or inhibit, directly or indirectly, the activity or the expression of PLA2R1 receptor.

9. Composition for use a medicament according to anyone of claims 7 to 8 which further comprise at least one other therapeutic agent useful for the prevention and/or the treatment of diabetes, and preferably of type-2 diabetes.

10. Pharmaceutical composition comprising at least one inhibitor of PLA2R1 and a pharmaceutically acceptable carrier.

11. Pharmaceutical composition according to claim 10 wherein said inhibitor is selected in the group consisting of of antisense oligonucleotides, or interfering RNAs, aptamers, ribozymes avoiding expression of PLA2R1 gene, antagonists of the PLA2R1 receptor activity or expression, antibodies or small molecules including polypeptides of less than 80 residues or pseudopeptides, which block or inhibit, directly or indirectly, the activity or the expression of PLA2R1 receptor.


SEQUENCE LISTING

<110>  UNIVERSITE CLAUDE BERNARD LYON 1
       INSTITUT NATIONAL DE LA SANTE ET DE LA RECHERCHE MEDICALE
       (INSERM)
       CENTRE LEON BERARD
       CENTRE NATIONAL DE LA RECHERCHE SCIENTIFIQUE (CNRS)

<120>  INHIBITION OF PLA2R1 FOR THE PREVENTION AND/OR THE TREATMENT OF
       TYPE-2 DIABETES

<130>  B72948 D36375

<160>  2

<170>  PatentIn version 3.5

<210>  1
<211>  4392
<212>  DNA
<213>  Homo sapiens



<220>
<221>  CDS
<222>  (1)..(4389)

<400>  1
atg ctg ctg tcg ccg tcg ctg ctg ctg ctg ctg ctg ctg ggg gcg ccg        48
cgg ggc tgc gcc gag ggt gtg gcg gcg gcg ctt acc ccc gag cgg ctc        96
ctg gag tgg cag gat aaa gga ata ttt gtt atc caa agt gag agt ctc       144
aag aaa tgc att caa gca ggt aaa tcg gtt ctg acc ctg gag aac tgc       192
aag caa gca aac aag cac atg ctg tgg aaa tgg gtt tca aac cat ggc       240
ctc ttt aac ata gga ggc agc ggt tgc ctg ggc ctg aat ttc tcc gcc       288
cca gag cag cca tta agc tta tat gaa tgt gac tcc acc ctc gtt tcc       336
tta cgg tgg cgc tgt aac agg aag atg atc aca ggc ccg ctg cag tac       384
tct gtc cag gtg gcg cat gac aac aca gtg gtg gcc tca cgg aag tat       432
att cat aag tgg att tct tat ggg tca ggt ggt gga gac att tgt gaa     480
tat cta cac aaa gat ttg cat aca atc aaa ggg aac acc cac ggg atg     528
ccg tgt atg ttt ccc ttc cag tat aac cat cag tgg cat cat gaa tgt     576
acc cgt gaa ggt cgg gaa gat gac tta ctg tgg tgt gcc acg aca agc     624
cgt tat gaa aga gat gaa aag tgg gga ttt tgc cct gat ccc acc tct     672
gca gaa gta ggt tgt gat act att tgg gag aag gac ctc aat tca cac     720
att tgc tac cag ttc aac ctg ctt tca tct ctc tct tgg agt gag gca     768
cat tct tca tgc cag atg caa gga ggt acg ctg tta agt att aca gat     816
gaa act gaa gaa aat ttc ata agg gag cac atg agc agt aaa aca gtg     864
gag gtg tgg gtg ggc ctc aat cag ctg gat gaa gac gct ggc tgg cag     912
tgg tct gat gga acg ccg ctc aac tat ctg aat tgg agc cca gag gta     960
aat ttt gag cca ttt gtt gaa gat cac tgt gga aca ttt agt tca ttt     1008
atg cca agt gcc tgg agg agt cgg gat tgt gag tcc acc ttg cca tat     1056
ata tgt aaa aaa tat cta aac cac att gat cat gaa ata gtt gaa aaa     1104
gat gcg tgg aaa tat tat gct acc cac tgt gag cct ggc tgg aat ccc     1152
tac aat cgt aat tgc tac aaa ctt cag aaa gaa gaa aag acc tgg cat     1200
gag gct ctg cgt tct tgt cag gct gat aac agt gca tta ata gac ata      1248
acc tca tta gca gag gtg gag ttt ctt gta acc ctc ctt gga gat gaa      1296
aat gca tca gaa aca tgg att ggt ttg agc agc aat aaa att cca gtt      1344
tcc ttt gaa tgg tct aat gac tct tca gtc atc ttt act aat tgg cac      1392
aca ctt gag ccc cac att ttt cca aat aga agc cag ctg tgt gtc tca      1440
gca gag cag tct gag gga cac tgg aaa gtc aaa aat tgt gaa gaa aga      1488
ctt ttt tac att tgt aaa aaa gca ggc cat gtc ctc tct gat gct gaa      1536
tca gga tgt caa gag gga tgg gag aga cat ggt gga ttc tgt tac aaa      1584
att gac aca gtc ctt cga agc ttt gac caa gct tcc agc ggt tat tac      1632
tgt cct cct gca ctt gta acc att aca aac agg ttt gaa cag gct ttt      1680
att acc agt ttg atc agt agt gtg gta aaa atg aag gac agt tat ttt      1728
tgg ata gct ctt cag gac caa aat gat acg gga gaa tac act tgg aag      1776
cca gta ggg cag aaa ccc gag ccg gtg cag tac aca cac tgg aac aca      1824
cac cag ccg cgc tac agt ggt ggc tgt gtt gcc atg cga gga agg cat      1872
cca ctt ggt cgc tgg gaa gtg aag cac tgt cgg cac ttt aag gca atg      1920
tcc ttg tgc aag cag cca gtt gaa aat cag gaa aaa gca gag tat gaa      1968
gag aga tgg ccc ttt cac ccc tgc tat ttg gac tgg gag tca gag cct      2016
ggt ctg gcc agt tgc ttc aag gta ttt cat agt gaa aaa gtt ctg atg      2064
aaa aga aca tgg aga gaa gct gaa gca ttt tgc gaa gaa ttt gga gct      2112
cat ctt gca agc ttt gcc cat att gag gaa gag aat ttt gtg aat gag      2160
ctc tta cat tca aaa ttt aat tgg aca gaa gaa agg cag ttc tgg att      2208
gga ttt aat aaa aga aac cca ctg aat gcc ggc tca tgg gag tgg tct      2256
gat aga act cct gtt gtc tct tcg ttt tta gac aac act tat ttt gga      2304
gaa gat gca aga aac tgt gct gtt tat aag gca aac aaa aca ttg ctg      2352
ccc tta cac tgt ggt tcc aaa cgt gaa tgg ata tgc aaa atc cca aga      2400
gat gtg aaa ccc aag att ccg ttc tgg tac cag tac gat gta ccc tgg      2448
ctc ttt tat cag gat gca gaa tac ctt ttt cat acc ttt gcc tca gaa      2496
tgg ttg aac ttt gag ttt gtc tgt agc tgg ctg cac agt gat ctt ctc      2544
aca att cat tct gca cat gag caa gaa ttc atc cac agc aaa ata aaa      2592
gcg cta tca aag tat ggt gca agt tgg tgg att gga ctt caa gaa gaa      2640
aga gcc aat gat gaa ttt cgc tgg aga gat gga aca cca gtg ata tac      2688
cag aac tgg gac aca gga aga gaa aga act gtg aat aat cag agc cag 2736
aga tgt ggc ttt att tct tct ata aca gga ctc tgg ggt agt gaa gag 2784
tgt tca gtt tct atg cct agt atc tgt aag cga aaa aag gtt tgg ctc 2832
ata gag aaa aag aaa gat aca cca aaa caa cat gga acg tgt ccc aaa 2880
gga tgg cta tat ttt aac tat aag tgc ctt ctg ctg aat atc ccc aaa 2928
gac cca agc agt tgg aag aac tgg acg cat gct caa cat ttc tgt gct 2976
gaa gaa ggg ggg acc ctg gtc gcc att gaa agt gag gtg gag caa gct 3024
ttc att act atg aat ctt ttt ggc cag acc acc agt gtg tgg ata 3069
ggt tta caa aat gat gat tat gaa aca tgg cta aat gga aag cct 3114
gtg gta tat tct aac tgg tct cca ttt gat ata ata aat att cca 3159
agt cac aat acc act gaa gtt cag aaa cac att cct ctc tgt gcc 3204
tta ctc tca agt aat cct aat ttt cat ttc act gga aaa tgg tat 3249
ttt gaa gac tgt gga aag gaa ggc tat ggg ttt gtt tgt gaa aaa 3294
atg caa gat act tct gga cac ggt gta aat aca tct gat atg tat 3339
cca atg ccc aat acc tta gaa tat gga aac aga act tac aaa ata 3384
att aat gca aat atg act tgg tat gca gca ata aaa acc tgc ctg 3429
atg cac aaa gca caa ctg gtc agc atc aca gac cag tat cac cag 3474
tcc ttc ctc act gtt gtc ctc aac cgg cta gga tat gcc cac tgg 3519
att gga ctg ttc acc aca gat aat ggt ctt aat ttt gac tgg tct 3564
gat ggc acc aaa tct tct ttc act ttt tgg aaa gat gag gag tcc 3609
tcc ctc ctt ggt gac tgc gtt ttt gcc gac agc aac gga cgc tgg 3654
cat agc aca gcc tgc gag tca ttt ctg caa ggt gcc att tgt cat 3699
gtg cca cct gaa aca aga caa tct gaa cac cca gag ttg tgc tca 3744
gaa aca tct att ccc tgg ata aaa ttt aaa agt aat tgc tac agt 3789
ttt tct aca gtc cta gac agt atg agt ttt gag gct gct cat gaa 3834
ttt tgc aaa aag gaa ggt tct aat ctt tta aca atc aag gat gag 3879
gct gaa aat gca ttt ctc cta gaa gag ctg ttt gct ttt ggt tct 3924
tct gtc cag atg gtt tgg ttg aat gct caa ttt gat ggt aac aat 3969
gaa acc ata aag tgg ttt gat gga act ccc aca gac cag tca aac 4014
tgg ggc att cgg aag cca gac aca gac tac ttc aag ccc cat cat 4059
tgt gtt gcc ttg agg atc cct gaa gga tta tgg cag cta tcc ccg 4104
tgt caa gaa aaa aag ggc ttt ata tgt aaa atg gag gca gat att 4149
cac act  gca gag gcg ctg cca  gaa aaa gga cca agt  cac agc atc        4194
att cct  ctt gcg gtt gta ctg  aca ctg ata gtc att  gtg gcc att        4239
tgc aca  ctt tcc ttc tgc ata  tac aag cat aac ggt  ggc ttc ttc        4284
agg aga  ctt gca ggg ttt cgg  aat cct tac tat cct  gca acc aac        4329
ttt agt  aca gta tat tta gaa  gaa aat att ctc att  tct gat ctt        4374
gag aag  agt gac caa taa                                             4392


<210>  2
<211>  1463
<212>  PRT
<213>  Homo sapiens

<400>  2